# EUROPEAN PATENT APPLICATION

(11) **EP 2 075 307 A2**
(43) Date of publication of application: **01.07.2009**
(21) Application number: 08021923.1
(22) Date of filing: 17.12.2008
(51) Int. Cl.: C09K 11/06

(54) **Organic electroluminescence material and element using the same**

(30) Priority: 25.12.2007 JP 2007332577
(71) Applicant: Yamagata Promotional Organization for Industrial Technology, Yamagata-shi, Yamagata 990-2473 (JP)
(72) Inventor: Oda, Atsushi, Yamagata-shi Yamagata 990-2743 (JP); Takayuki, Horiuichi, Yamagata-shi Yamagata 990-2743 (JP); Kimura, Masato, Yamagata-shi Yamagata 990-2743 (JP); Tanaka, Junich, Yamagata-shi Yamagata 990-2743 (JP)
(74) Representative: HOFFMANN EITLE

(57) **Abstract**

An organic EL element having one or a plurality of organic layers including a light emitting layer between a pair of electrodes is arranged such that at least one layer of the above-mentioned organic layers contains a compound as expressed by the following general formula (1) independently or as a mixture. 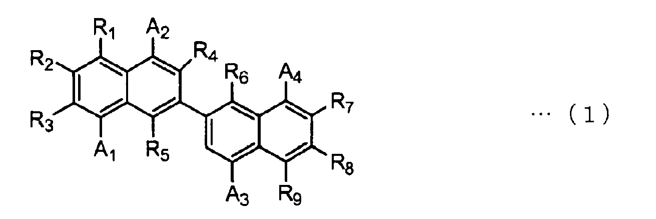 (where R₁-R₉ are selected from the group consisting of hydrogen, an alkyl group, a cycloalkyl group, an alkoxy group, a cycloalkoxy group, and an aryloxy group, and they may be the same groups or the groups different from one another, and A₁-A₄ are selected from the group consisting of a phenyl group which is either substituted or unsubstituted and a 5 or 6 member heterocyclic ring group which is either substituted or unsubstituted, and they may be the same groups or the groups different from one another.)

## Description

### BACKGROUND OF THE INVENTION

### Field of the Invention

The present invention relates to an organic electroluminescence material made of a novel naphthalene derivative for obtaining blue luminescence excellent in color purity, and to an organic electroluminescence element (hereinafter referred to as organic EL element) using the same.

### Description of the Related Art

Since the organic EL element is a self-luminescence type element which includes an organic compound as a light emitting material and allows luminescence at a high speed, it is suitable for displaying a video image, and it has features that allow an element structure to be simple and a display panel to be thin. Having such outstanding features, the organic EL element is spreading in everyday life as a cellular phone or a vehicle-mounted display.

Further, unlike LED, taking advantage of the features that it allows planar luminescence and is thin and lightweight, technical development as an illumination panel light source has also been carried out.

The above-mentioned organic EL element has been improved in luminescence efficiency by doping a host with a very small quantity of light emitting colorant. For this reason, the host material is an important material in order to obtain an efficient organic EL element.

In particular, in order to obtain a white light emitting element with high color rendering properties, the host material which can efficiently take blue luminescence from the blue-light emitting colorant is indispensable.

As the host materials for emitting blue light are conventionally known. For example, anthracene derivatives as shown in the following [Chemical Formula 1] and [Chemical Formula 2], a distyrylarylene derivative as shown in the following [Chemical Formula 3], a naphthalene derivative as shown in the following [Chemical Formula 4], etc. are known (for example, Japanese Patent Application Publication No. H11-312588, Japanese Patent Application Publication No. 2005-222948, Japanese Patent Application Publication No. H2-247278, and J. Shi, et al., Applied Physics Letters, 80 (17), p.3201).

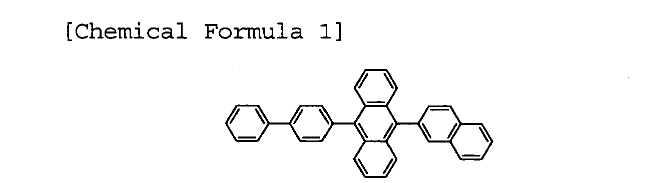

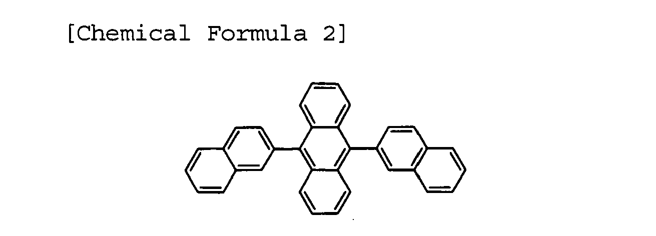

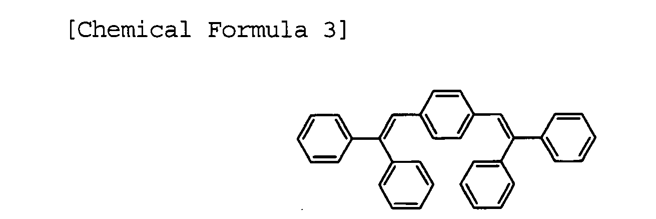

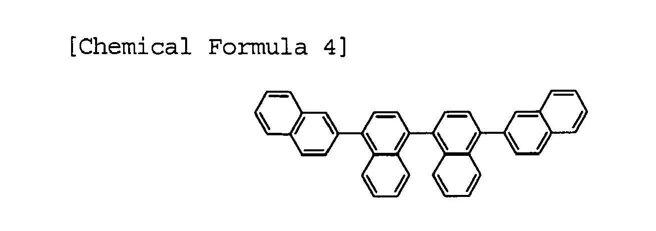

However, the materials as disclosed in the above-mentioned Japanese Patent Application Publication No. H11-312588, Japanese Patent Application Publication No. 2005-222948, Japanese Patent Application Publication No. H2-247278, and J. Shi, et al. , and Applied Physics Letters, 80 (17), p. 3201 emit lights of bluish green and light blue. In the case where they are used as a host material, it is difficult to obtain blue luminescence excellent in color purity, which is required for the white light emitting element with high color rendering properties.

### SUMMARY OF THE INVENTION

The present invention arises in order to solve the above-mentioned technical problem, and aims at providing a new organic electroluminescence material which emits blue light excellent in color purity and an organic EL element using the same.

The organic EL material in accordance with the present invention is expressed by the following general formula (1).

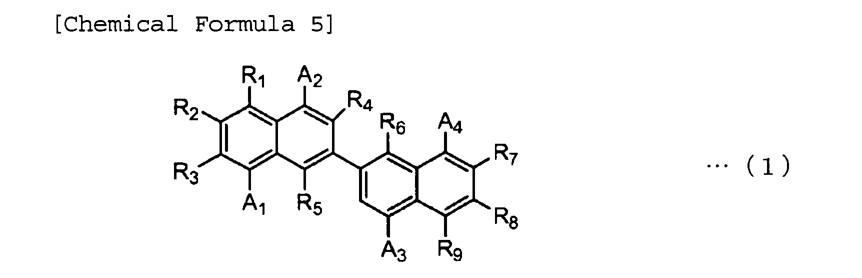

In the above-mentioned general formula (1), R₁-R₉ are selected from the group consisting of hydrogen, an alkyl group, a cycloalkyl group, an alkoxy group, a cycloalkoxy group, and an aryloxy group, and they may be the same groups or the groups different from one another. A₁-P₄ are selected from the group consisting of a phenyl group which is either substituted or unsubstituted and a 5 or 6 member heterocyclic ring group which is either substituted or unsubstituted, and they may be the same groups or the groups different from one another.

By using such a compound as the organic EL material, it is possible to obtain blue luminescence which is useful as a luminescence band and excellent in color purity.

Further, the organic EL element in accordance with the present invention is an organic EL element having one or a plurality of organic layers in which a light emitting layer is provided between a pair of electrodes, characterized in that at least one layer of the above-mentioned organic layers contains the above-mentioned organic EL material independently or as a mixture.

By using the above-mentioned organic EL material in accordance with the present invention, it is possible to construct an element which emits blue light excellent in color purity.

In the above-mentioned organic EL element, it is preferable that at least one layer of the above-mentioned organic layers is a light emitting layer containing the above-mentioned organic EL material as a host material and a fluorescence or phosphorescence emitting material as a guest material.

Further, as for the above-mentioned electrode, it is preferable that a transparent electroconductive thin-film is formed on a transparent substrate.

As described above, according to the organic EL material in accordance with the present invention, the blue luminescence excellent in color purity is obtained. Thus, by using this, it is possible to provide the white-light emitting element with high color rendering properties.

Therefore, it is expected that the organic EL element in accordance with the present invention would be applied to flat panel displays for OA computers and flat TV sets which nowadays require better color reproducibility, a light source for an illumination apparatus, a light source for a copying machine, light sources which take advantage of planar light-emitting members, such as backlight sources for a liquid crystal display, meters, etc., a display board, and a beacon light.

### BRIEF DESCRIPTION OF THE DRAWINGS

Fig. 1 is a sectional view schematically showing a layer structure of an organic EL element with respect to Example 2.
Fig. 2 is a graph showing a light emission spectrum of the organic EL element with respect to Example 2.
Fig. 3 is a graph showing a light emission spectrum of the organic EL element with respect to Comparative Example 1.

### DESCRIPTION OF THE PREFERRED EMBODIMENTS

Hereafter, the present invention will be described in detail.

An organic EL material in accordance with the present invention is a compound expressed by the above-mentioned general formula (1).

Such a binaphthyl derivative is a novel compound which emits blue light excellent in color purity. By using this, it is possible to provide the white light emitting element with high color rendering properties.

In the above-mentioned general formula (1), R₁-R₉ are selected from the group consisting of hydrogen, an alkyl group, a cycloalkyl group, an alkoxy group, a cycloalkoxy group, and an aryloxy group, and they may be the same groups or the groups different from one another. Further, A₁-A₄ are selected from the group consisting of a phenyl group which is either substituted or unsubstituted and a 5 or 6 member heterocyclic ring group which is either substituted or unsubstituted, and they may be the same groups or the groups different from one another.

Among the above-mentioned substitution groups, the alkyl group indicates a saturated aliphatic hydrocarbon group, such as for example a methyl group, an ethyl group, a propyl group, a butyl group, etc., and it may be of a straight chain or may be of a branched chain.

The cycloalkyl group indicates a saturated alicyclic hydrocarbon group, such as for example, a cyclohexyl group, a norbornyl group, an adamantyl group, etc., and they may be either unsubstituted or substituted.

The alkoxy group indicates, for example, a saturated aliphatic hydrocarbon group through ether bonds, such as a methoxy group etc. , and it may be of a straight chain, or may be of a branched chain.

The cycloalkoxy group indicates a cyclic saturated aliphatic hydrocarbon group through ether bonds, such as for example, a cyclohexyl group etc., and it may be either unsubstituted or substituted.

The aryloxy group indicates an aromatic hydrocarbon group through ether bonds, such as for example a phenoxy group etc., and the aromatic hydrocarbon group may be either unsubstituted or substituted.

The substituted phenyl group indicates a phenyl group substituted with an alkyl group, a cycloalkyl group, an alkoxy group, a cycloalkoxy group, or an aryloxy group.

The heterocyclic group indicates a group which contains either nitrogen and sulfur or oxygen as a ring constituent element in addition to carbon. As examples thereof, there may be mentioned oxazole, oxadiazole, thiazole, thiadiazole, furan, furazan, thiophene, pyrane, thiopyrane, pyrrole, pyrazole, imidazoline, imidazole, pyrazine, pyridine, pyridazine, pyrimidine, triazole, triazine, etc., and they may be either unsubstituted or substituted.

Hereafter, among the compounds expressed by the above-mentioned general formula (1), particular examples of the substitution group combined with any of A₁-A₄ will be mentioned. In addition, in a phenyl group which is shown in the following [Chemical Formula 6] and is either substituted or unsubstituted and a 5 or 6 member heterocyclic ring group which is either substituted or unsubstituted, "X" indicates positions bonded with a naphthalene ring, i.e., bonding positions in A₁-A₄.

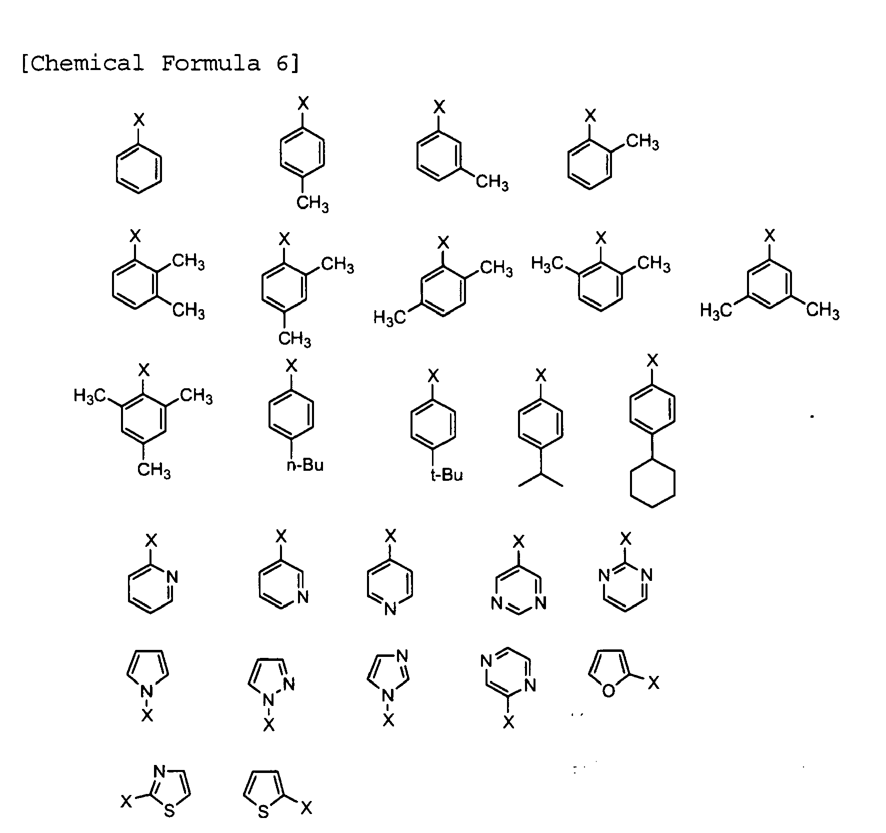

Furthermore, among the compounds expressed by the above-mentioned general formula (1), a structure of a particular compound is illustrated below.

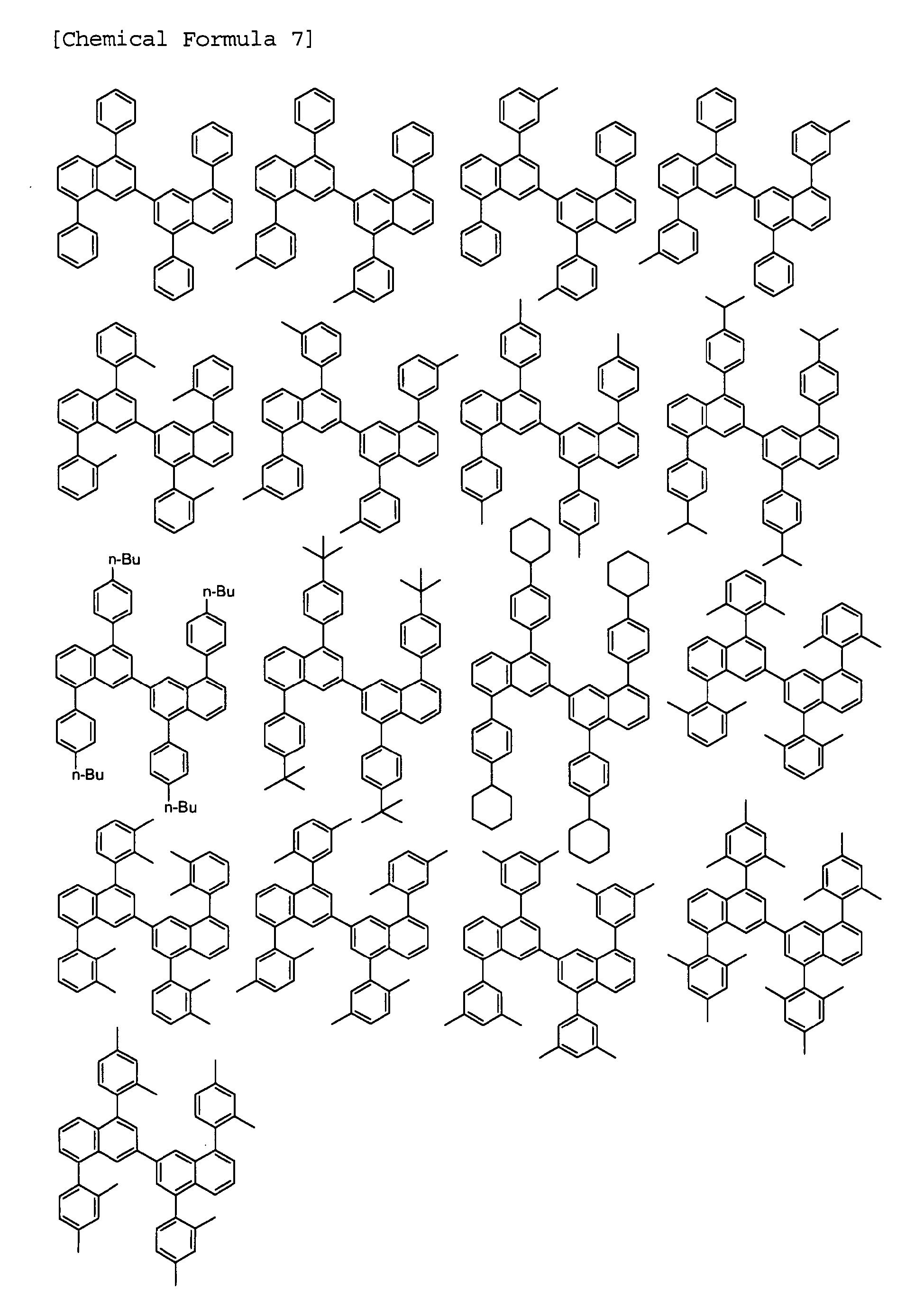

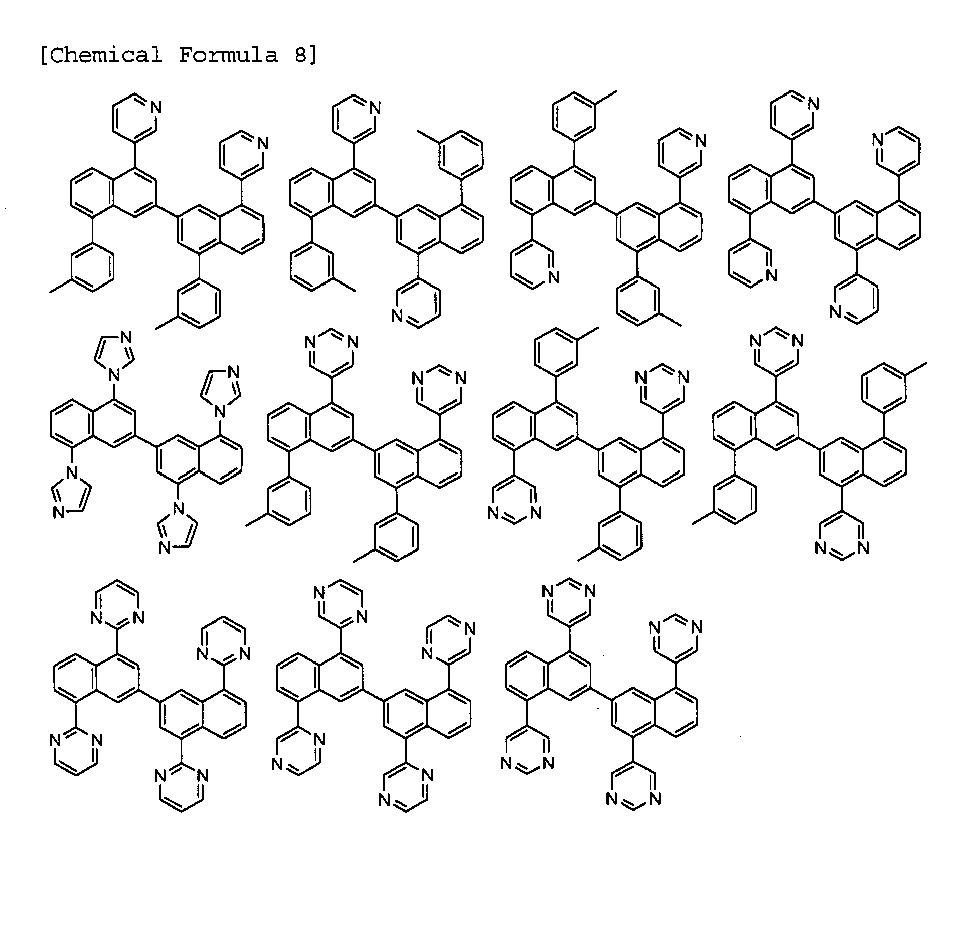

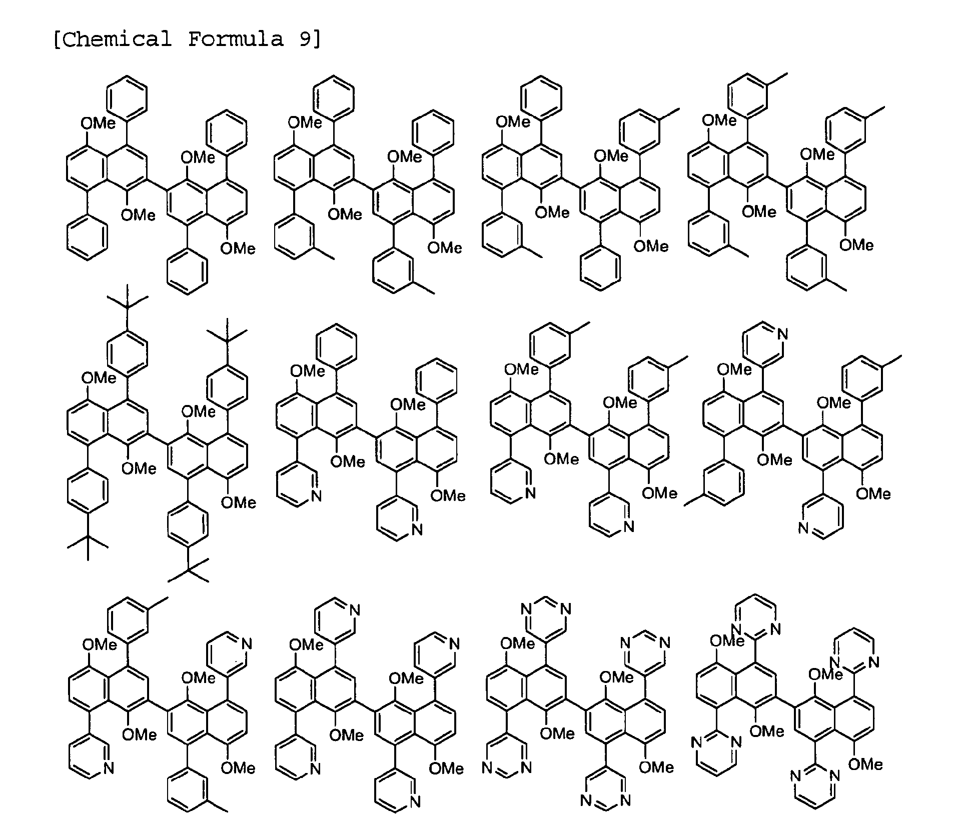

Among the compounds illustrated in the above-mentioned [Chemical Formula 7] through [Chemical Formula 9], there may be particularly mentioned, as a representative, 4,4', 8,8'-tetra (3-methylphenyl)-2,2'-binaphthyl (hereinafter abbreviated to TMBN) as expressed in the following [Chemical Formula 10].

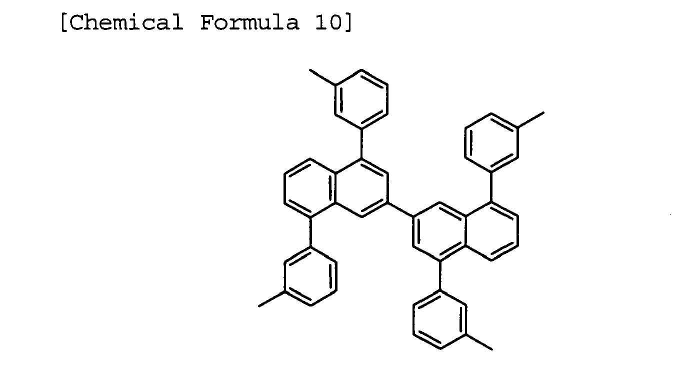

The compound expressed by the above-mentioned general formula (1) can be synthesized by way of a conventionally known synthetic reaction.

For example, diamino-substituted naphthalene is used as a material, and an amino group is substituted by a predetermined halogen group by way of the Sandmeyer reaction. As for the thus obtained di-halogen-substituted derivative, meta positions of the halogen substitution group are brominated in the presence of transition metal powder (preferably iron powder), to obtain a bromine derivative.

Then, by way of the coupling reaction using transition metal catalysts, such as Ni, Pd, etc., a dimer is obtained in which the naphthalene rings are connected each other at the meta positions. Furthermore, the naphthalene derivative expressed by the above-mentioned general formula (1) is synthesized by way of the coupling reaction between this dimer and a corresponding cross coupling agent using the transition metal catalysts, such as Ni, Pd, etc.

At this time, by suitably selecting a halogen group at the time of the Sandmeyer reaction, it is possible to obtain reaction selectivity. It is also possible to perform previously the coupling reaction with the corresponding cross coupling agent using the transition metal catalysts, such as Ni, Pd, etc., then to carry out dimerization, thus synthesizing the naphthalene derivative expressed by the above-mentioned general formula (1). Further, by selecting the halogen group, the asymmetrical compound expressed by the above-mentioned general formula (1) can also be synthesized in the coupling reaction using the transition metal catalysts, such as Ni, Pd, etc.

In addition, the material is not particularly limited, but a dihydroxy derivative, a dialkoxy derivative, etc. can also be used other than the above-mentioned diamino derivative of naphthalene.

Further, the compound expressed by the above-mentioned general formula (1) can also be synthesized by way of the Diels-Alder reaction.

The organic EL element in accordance with the present invention provided with the layer containing the above-mentioned organic EL material which can obtain blue luminescence excellent in color purity has a structure in which one or a plurality of organic layers are laminated between electrodes. As particular examples thereof, there may be mentioned a structure having "first electrode / light emitting layer / second electrode", "first electrode / hole transport layer / light emitting layer / electron transport layer / second electrode", "first electrode / hole transport layer / light emitting layer / second electrode", "first electrode / light emitting layer / electron transport layer / second electrode", etc.

Furthermore, it is also possible to employ the known lamination structure further including a hole injection layer, a hole transport light emitting layer, an electron injection layer, an electron transport light emitting layer, etc.

As for the electrode of the organic EL element in accordance with the present invention, it is preferable that the transparent electroconductive thin-film is formed on the transparent substrate.

The above-mentioned substrate is a support member of the organic EL element. In the case where the substrate side is a light emitting side, it is preferable to use a transparent substrate which is transmissive in visible light. It is preferable that the optical transmissivity is 80% or more. More preferably, it is 85% or more. Most preferably, it is 90% or more.

In general, the above-mentioned transparent substrate employs glass substrates made of, such as for example, optical glass (BK7, BaK1, F2, etc.), silica glass, non alkali glass, borosilicate glass, aluminosilicate glass, polymer substrate made of, such as for example, acrylic resins (PMMA, etc.), polycarbonate, polyether sulphonate, polystyrene, polyolefin, an epoxy resin, and polyethylene terephthalate, polyester, etc.

Although the above-mentioned substrate having a thickness of approximately 0.1-10 mm is usually used, it is preferable that the thickness is 0.3-5 mm in view of mechanical strength, weight, etc. More preferably it is 0.5-2 mm.

Further, in the present invention, it is preferable that the first electrode is provided on the above-mentioned substrate. Although this first electrode is usually an anode and is made of a metal, an alloy, an electroconductive compound, etc., with a large work function (4 eV or more), it is preferable to be formed as a transparent electrode on the above-mentioned transparent substrate.

In general, metal oxides, such as indium tin oxide (ITO), indium zinc oxide, zinc oxide, etc. are used for this transparent electrode. In particular, ITO is preferably used in terms of its transparency, conductivity, etc.

In order to secure the transparency and conductivity, it is preferable that a film thickness of this transparent electrode is 80-400 nm. More preferably, it is 100-200 nm.

It is preferable that the anode is usually formed by way of a sputtering method, a vacuum deposition method, etc., and formed as the transparent electroconductive thin-film.

On the other hand, in the case where the above-mentioned anode is the first electrode, a cathode of the second electrode which faces this anode is made of a metal, an alloy, and a conductive compound having a small work function (4eV or less). As examples thereof, there may be mentioned aluminum, an aluminum-lithium alloy, a magnesium-silver alloy, etc.

It is preferable that the film thickness of the above-mentioned cathode is 10-500 nm. More preferably, it is 50-200 nm.

The above-mentioned anode and cathode can be formed by forming a film by way of methods usually used, such as a sputtering method, an ion plating method, a vapor-depositing method, etc.

Respective materials used for the above-mentioned hole injection layer, hole transport layer, and hole transport light emitting layer are not particularly limited, but can be suitably selected from known materials to use.

In particular, they may be bis(di(p-trill)aminophenyl)-1,1-cyclohexane (common name: TAPc), Spiro-TPD [Chemical Formula 11], N,N'-diphenyl-N,N'-bis(3-methylphenyl)-1,1'-biphenyl- 4,4'-diamine (common name: TPD),
N,N'-diphenyl-N,N'-bis(1-naphthyl)-1,1'-biphenyl-4,4'-diamine (common name: alpha-NPD), TPTE [Chemical Formula 12], and arylamine derivatives such as starburst amines [Chemical Formula 13], styrylamines [Chemical Formula 14], etc.

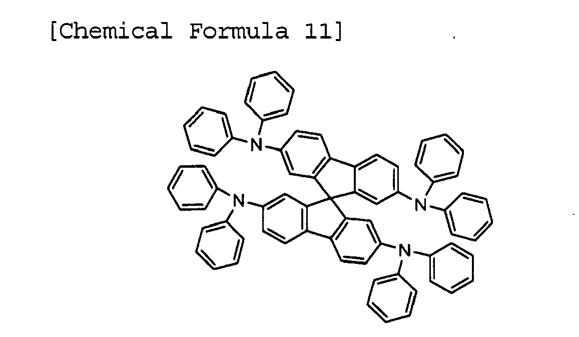

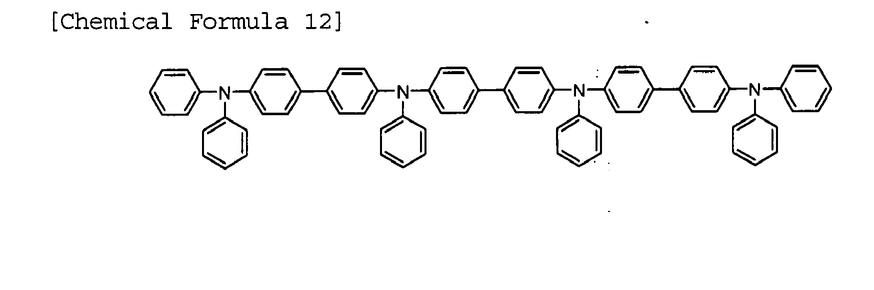

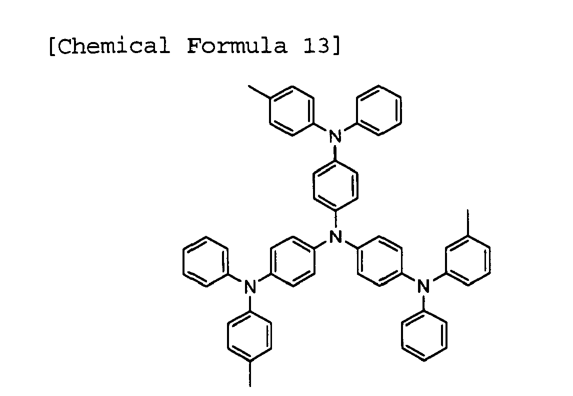

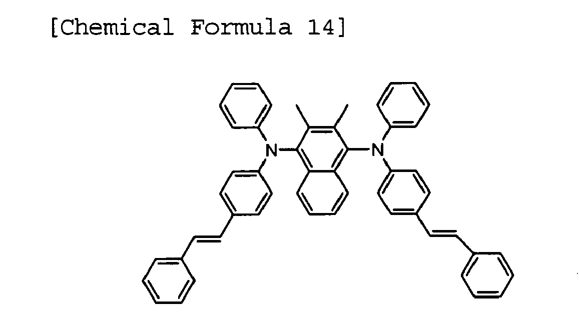

Further, it is also possible to use carbazole derivatives, such as bis(N-arylcarbazole) [Chemical Formula 15], bis(N-alkenylcarbazole), bis(N-alkylcarbazole), etc., a pyrazoline derivative, , and styryl compounds and their derivatives, such as [Chemical Formula 16], [Chemical Formula 17], [Chemical Formula 18], etc.

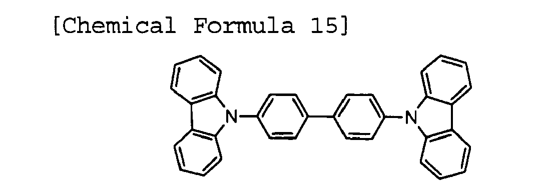

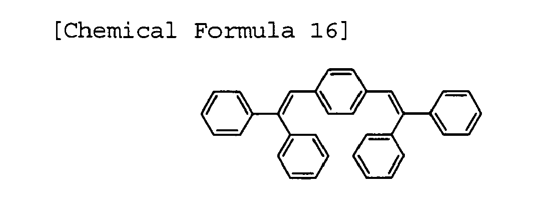

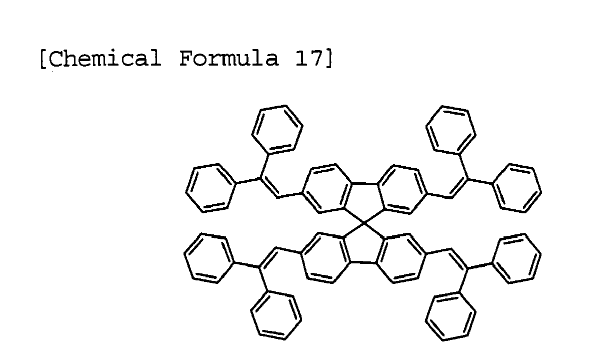

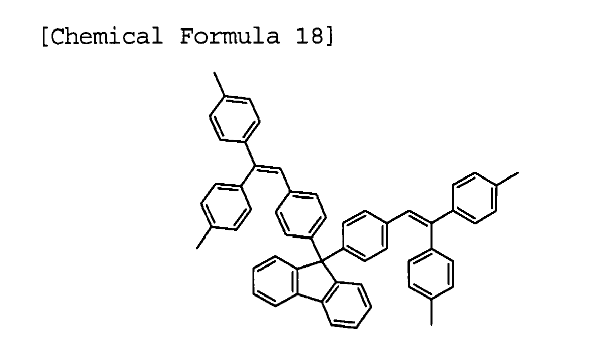

Alternatively, it is also possible to use fused multi-ring aromatic hydrocarbon compounds and those derivatives, such as anthracene, triphenylene, perylene, naphthalene, pyrene, coronene, chrysene, naphthacene, tetracene, phenanthrene, etc. as well as multi-ring compounds and those derivatives, such as paraterphenyl, quaterphenyl, m-phenylenes [Chemical Formula 19], [Chemical Formula 20], etc.

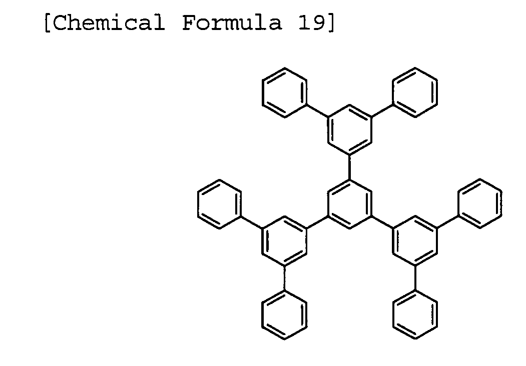

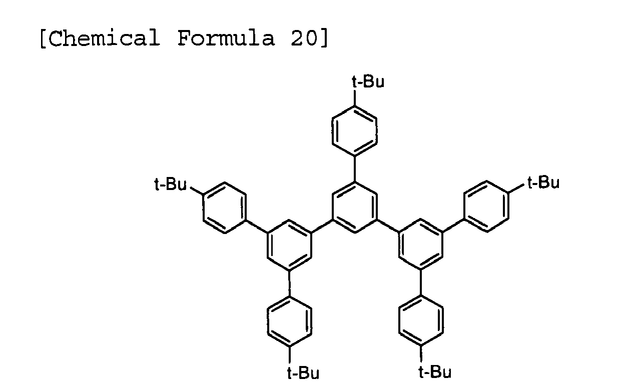

Furthermore, the hole injection layer, the hole transport layer, and the hole transport light emitting layer may employ the above-mentioned organic compound which is dispersed in a polymer, an oligomer, or a dendrimer, or which is polymerized, oligomerized or dendrimerized.

Still further, it is also possible to use so-called π-conjugated polymers etc., such as polyparaphenylenevinylene, polyfluorene, their derivatives, etc., a hole transport disconjugate polymer represented by poly(N-vinylcarbazole), σ-conjugated polymers represented by polysilanes, etc. Furthermore, so-called conjugated oligomers, such as a fluorene oligomer and its derivative, etc. can also be used.

Further, besides the above-mentioned materials, the hole injection layer may employ conductive polymers, such as metal phthalocyanines, non-metal phthalocyanines, carbon film, fluorocarbon film, polystyrene sulfonic acid (PEDOT-PSS), polyaniline, etc.

Furthermore, the above-mentioned organic compound may be reacted with organic oxidizing dopants, such as tetracyanoquinodimethane, trinitrofluorenone, etc., and inorganic oxidizing dopants, such as vanadium oxide, molybdenum oxide, tungstic oxide, aluminum oxide, etc., to form a radical cation, and can be used as the hole injection transport layer. Although the oxidizing dopant concentration in this hole injection transport layer is not particularly limited, but it is preferable to be approximately 0.1 - 99 % by weight.

Yet further, respective materials used for the electron injection layer, the electron transport layer, and the electron transport light emitting layer are not particularly limited either, but can be suitably selected from the known materials to use.

As particular examples, there may be mentioned multi-ring compounds and those derivatives, such as paraterphenyl, quaterphenyl, m-phenylenes [Chemical Formula 19], [Chemical Formula 20], etc., and styryl compounds and those derivatives, such as [Chemical Formula 16], [Chemical Formula 17], [Chemical Formula 18], etc.

Further, it is also possible to use fused multi-ring aromatic hydrocarbon compounds and those derivatives, such as anthracene, triphenylene, perylene, naphthalene, pyrene, coronene, chrysene, naphthacene, tetracene, phenanthrene, etc., as well as fused heterocyclic compounds and those derivatives, such as phenanthroline, bathophenanthroline, bathocuproin, phenanthridine, acridine, quinolin, quinoxaline, pyridine [Chemical Formula 21], pyrimidine, pyrrole, pyrazole, pyridazine, pyrazine, phthalazine, naphthylidine, quinazoline, cinnoline, thiazole, oxadiazole, oxazole, triazine, phenazine, imidazole, benzoxazole, benzothiazole, benzoimidazole, triazole, porphyrin, etc.

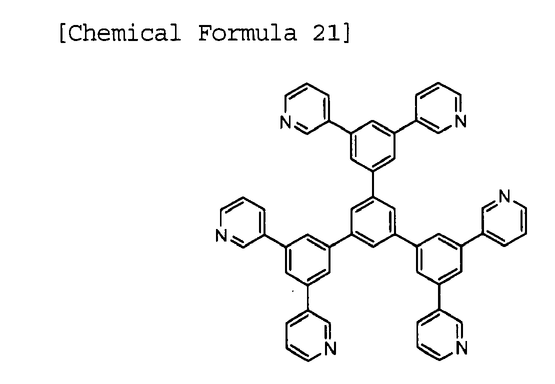

Further, it is also possible to use metal chelate complex materials, for example, a quinolinol aluminum complex, a benzoxazole zinc complex, a benzothiazole zinc complex, an azomethine zinc complex, a europium complex, an iridium complex, a platinum complex, etc., in which Al, Zn, Be, Ir, Pt, Tb, Eu, etc. are provided as central metals, and oxadiazole, thiadiazole, phenylpyridine, and quinolin structures are provided as ligands.

It is also possible to use organosilicon compounds and those derivatives, such as silole, siloxane, etc., organic boron compounds and those derivatives, such as triarylborane etc., and pentavalent phosphorus compounds and those derivatives etc., such as triarylphosphoxide etc.

Furthermore, the electron injection layer, the electron transport layer, and the electron transport light emitting layer may employ the above-mentioned organic compound which is dispersed in a polymer, an oligomer, or a dendrimer, or which is polymerized, oligomerized or dendrimerized.

Further, it is also possible to use so-called π-conjugated polymers, such as polyparaphenylenevinylene, polyfluorene, their derivatives, etc., and the electron transport disconjugate polymer etc. represented by polyvinyl oxadiazole. Furthermore, the so-called conjugated oligomers etc, such as a fluorene oligomer and its derivative, etc. can be used.

Besides the above-mentioned organic compounds, as the constituent material of the electron injection layer, it is possible to use single metals, such as Ba, Ca, Li, Cs, Mg, Sr, W, etc., metal fluorides, such as magnesium fluoride, calcium fluoride, strontium fluoride, barium fluoride, lithium fluoride, cesium fluoride, etc., metal alloys, such as an aluminum lithium alloy etc., metal oxides, such as magnesium oxide, strontium oxide, aluminum oxide, etc., and organometallic complexes, such as sodium polymethylmethacrylate polystyrene sulphonate etc.

Furthermore, the above-mentioned organic compound may be reacted with organic reducing dopants, such as 8-hydroxyquinolin Cs, Li organometallic complex, etc., to form a radical anion, which can be used as the electron injection transport layer.

Further, single metals, such as Ba, Ca, Li, Cs, Mg, Sr, W, etc., metal oxides, such as magnesium oxide, strontium oxide, aluminum oxide, etc., metal salts, such as magnesium fluoride, calcium fluoride, strontium fluoride, barium fluoride, lithium fluoride, cesium fluoride, cesium chloride, strontium chloride, etc., and inorganic reducing dopants may be mixed or dispersed to form a radical anion, which can be used as the electron injection transport layer.

Although the reducing dopant concentration in the above-mentioned electron injection transport layers is not particularly limited, it is preferable to be approximately 0.1 - 99 % by weight.

Further, it is possible to constitute the organic layer of the organic EL element in accordance with the present invention by using a bipolar material. By bipolar material is meant a material which can convey both the hole and the electron and may emit light by itself.

Respective materials used for the bipolar transport layer and a bipolar light emitting layer are not particularly limited.

As examples thereof, there may be mentioned styryl compounds and those derivatives, such as [Chemical Formula 16], [Chemical Formula 17], [Chemical Formula 18], etc., multi-ring aromatic compounds and those derivatives, such as paraterphenyl, quaterphenyl, m-phenylenes [Chemical Formula 19], [Chemical Formula 20] etc., fused multi-ring aromatic hydrocarbon compounds and those derivatives, such as anthracene, triphenylene, perylene, naphthalene, pyrene, coronene, chrysene, naphthacene, tetracene, phenanthrene, etc., carbazole derivatives [Chemical Formula 15], such as bis(N-arylcarbazole), bis(N-alkenylcarbazole), and bis(N-alkylcarbazole), fused heterocyclic compounds, such as thiophene etc.

Further, as examples other than these derivatives etc., there may be mentioned 4,4-bis(2,2-diphenyl-ethene-1-yl)diphenyl (DPVBi) [Chemical Formula 22], spiro6 [Chemical Formula 23], 2,2',7,7'-tetrakis(carbazole-9-yl)-9,9'-spiro-bifluorene [Chemical Formula 24], 4,4'-di(N-carbazolyl)-2',3',5',6'-tetraphenyl-p-terphenyl [Chemical Formula 25], 1,3-bis(carbazole)-9-yl-benzene [Chemical Formula 26], and 3-tert-butyl-9,10-di(naphtha-2-yl)anthracene (common name: TBADN) [Chemical Formula 27].

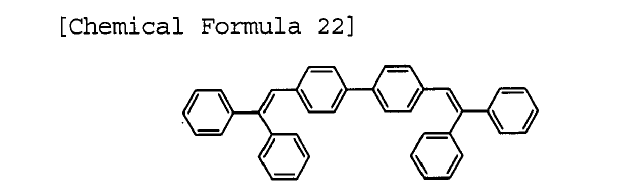

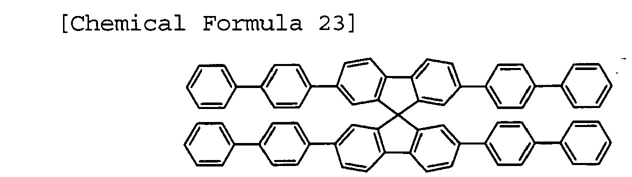

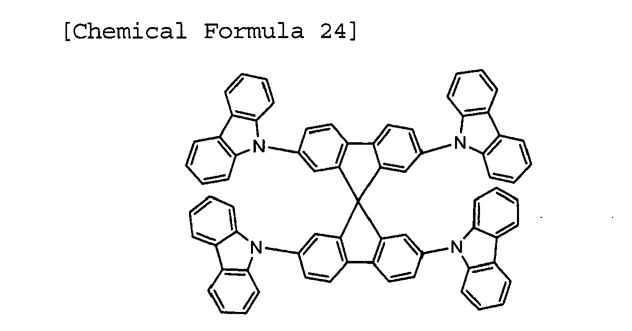

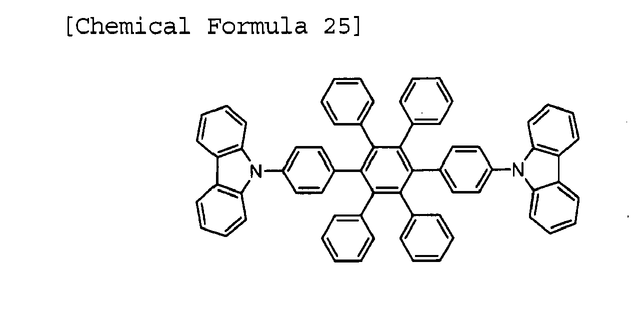

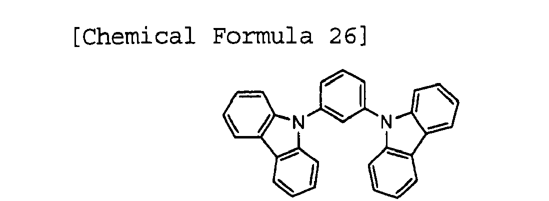

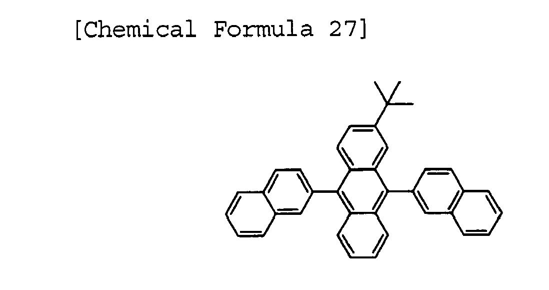

The bipolar material may employ the above-mentioned organic compound which is dispersed in a polymer, an oligomer, or a dendrimer, or which is polymerized, oligomerized or dendrimerized.

Further, it is also possible to use the so-called π-conjugated polymers, such as polyparaphenylene vinylene, polyfluorene, those derivatives, etc., and disconjugate polymers etc. represented by polyvinylcarbazole. Furthermore, so-called conjugated oligomers etc., such as a fluorene oligomer and its derivative, etc. can also be used.

Furthermore, copolymers, such as poly(vinyltriarylaminevinyloxadiazole) etc., where monomers having a hole transport function and an electron transport function exist in the same molecule, and a dendrimer can also be used.

Still further, it is possible to use the above-mentioned bipolar material which is reacted with the oxidizing dopant or reducing dopant as described above to form the hole injection layer or the electron injection layer. Especially, it is preferable that the oxidizing dopant is molybdenum oxide or vanadium oxide.

Although the organic EL material expressed by the above-mentioned general formula (1) can be used for the light emitting layer independently, it may be dispersed together with other hole transport material, light emitting material, electron transport material, etc., or doped, and can also be used combining with any of the above-mentioned organic layers.

In the organic EL element in accordance with the present invention, it is especially preferable that the above-mentioned blue luminescent material is used as a guest material to form the light emitting layer where it is included together with other host material. It is preferable that the concentration of the organic EL material expressed by the above-mentioned general formula (1) in this case is 0.1 - 99 % by weight. Further, it is possible to use it by combining with two or more types of other host materials.

In the case where the organic EL material expressed by the above-mentioned general formula (1) is used as the host material of the light emitting layer, the guest material of the light emitting layer may be a fluorescence or phosphorescence emitting material.

As examples thereof, there maybe mentioned multi-ring compounds and those derivatives, such as paraterphenyl, quaterphenyl, etc., distyrylaryl compounds and those derivatives, such as [Chemical Formula 16], [Chemical Formula 17], [Chemical Formula 18], etc., a tetraphenylbutadiene derivative, a pyrazoline derivative, an oxadiazole derivative, a coumarin derivative, a styrylamine derivative [Chemical Formula 14], fused multi-ring aromatic hydrocarbon compounds and those derivatives, such as anthracene [Chemical Formula 28], triphenylene, perylene, naphthalene [Chemical Formula 29], pyrene, coronene, chrysene, naphthacene, tetracene, phenanthrene, etc.

Further, it is also possible to use metal chelate complex materials, for example, a quinolinol aluminum complex, a benzoxazole zinc complex, a benzothiazole zinc complex, an azomethine zinc complex, a europium complex, a terbium complex, an iridium complex, a platinum complex, etc., in which Al, Zn, Be, Ir, Pt, Tb, Eu, etc. are provided as central metals, and oxadiazole, thiadiazole, phenylpyridine, and quinolin structures are provided as ligands. In particular, the metal chelate complexes represented by FIrpic [Chemical Formula 30) and those derivatives are mentioned.

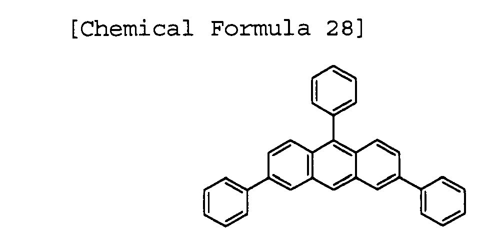

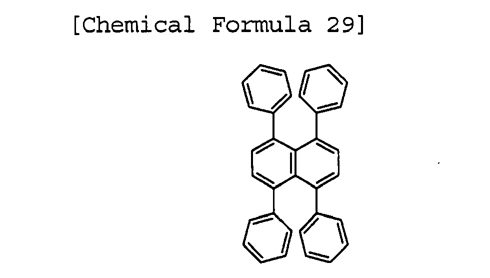

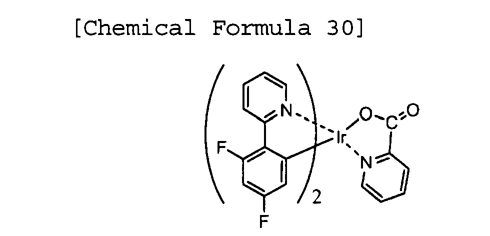

In addition, the light emitting layer may be made of the bipolar material as described above. The organic EL material expressed by the above-mentioned general formula (1) is included in the layer formed of the bipolar material independent or as a mixture, so that blue luminescence can also be obtained.

The bipolar material used for this light emitting layer may be a material which emits fluorescence or phosphorescence by itself. The bipolar material may be contained in any of the hole injection transport layer, the light emitting layer, and the electron injection transport layer.

Further, the material expressed by the above-mentioned general formula (1) can be used as the guest material, and the fluorescence or phosphorescence emitting material can also be used for the host material.

As examples of the host material in this case, there may be mentioned m-phenylene derivatives [Chemical Formula 20] and [Chemical Formula 21], ketone compounds and those derivatives, such as diarylketone etc., carbazole derivatives, such as bis(N-arylcarbazole) [Chemical Formula 16], bis(N-alkenylcarbazole), and bis(N-alkylcarbazole), etc., and an iridium complex represented by Ir(ppz)₃, [Chemical Formula 31).

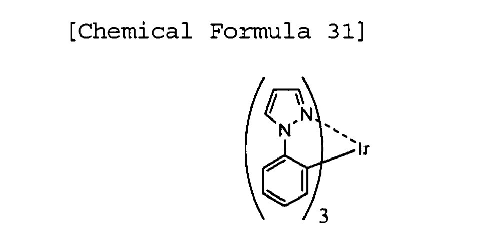

The above-mentioned host material may employ the above-mentioned organic compound which is polymerized, oligomerized or dendrimerized.

Further, it is also possible to use, for example, the so-called π-conjugated polymers, such as polyfluorene etc., and disconjugate polymers etc. represented by polyvinylcarbazole. Furthermore, so-called conjugated oligomers, such as a fluorene oligomer and its derivative, etc. can also be used.

The formation of each of the above-mentioned organic layers can be performed by way of dry processes, such as a vacuum deposition process, a sputtering process, etc., and wet processes,' such as an ink-jet process, a casting process, a dip coat process, a bar coat process, a blade coat process, a roll coat process, a photogravure coat process, a flexographic printing process, a spray coat process, etc. Preferably, the film formation is carried out by vacuum deposition.

Further, although a film thickness of each of the above-mentioned layers is suitably determined depending on its conditions in view of adaptability between the respective layers, the whole layer thickness to be required, etc., it is usually preferable to be within a range from 5 nm to 5 micrometers.

In the organic EL element provided with the layer containing the organic EL material expressed by the above-mentioned general formula (1) independently or as a mixture, the blue luminescence having high color purity and provided by the organic EL material expressed by the above-mentioned general formula (1) is combined with green and red luminescence so that white luminescence with high color-rendering properties may be obtained.

As a method of obtaining white luminescence with high color-rendering properties in particular, there may be mentioned a method of adding green luminescence and yellow to orange luminescence to the blue luminescence by the organic EL material expressed by the above-mentioned general formula (1) as complementary colors, a method of causing each of the blue, green, and red luminescent materials containing the material expressed by the above-mentioned general formula (1) to emit light independently, etc.

The above-mentioned green luminescent material or the red luminescent material may be a fluorescence or phosphorescence emitting material.

As examples thereof, there may be mentioned a quinacridone derivative, a squarylium derivative, a porphyrin derivative, a coumarin derivative, a dicyanopyrane derivative, arylamine compounds and those derivatives, such as anthracenediamine etc., fused multi-ring aromatic hydrocarbon compounds and those derivatives, such as perylene, rubrene, tetracene, decacyclene, etc., phenoxazone, a quinoxaline derivative, a carbazole derivative, and a fluorene derivative.

Further, it is also possible to use metal chelate complex materials, for example, a quinolinol aluminum complex, a benzoxazole zinc complex, a benzothiazole zinc complex, an azomethine zinc complex, a europium complex, a terbium complex, an iridium complex, a platinum complex, etc., in which Al, Zn, Be, Ir, Pt, Tb, Eu, etc. are provided as central metals, and oxadiazole, thiadiazole, phenylpyridine, and quinolin structures are provided as ligands. In particular, the metal chelate complexes represented by Ir(ppy)₃ [Chemical Formula 32], Irpiq₃ [Chemical Formula 33] and those derivatives are mentioned.

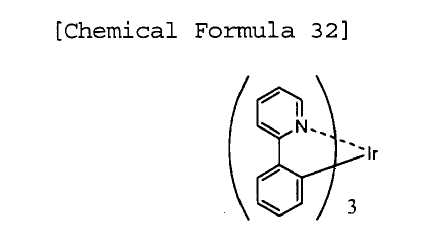

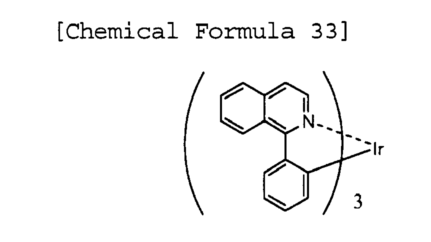

### [Examples]

Hereafter, the present invention will be described still more particularly with reference to Examples. However, the present invention is not limited to the following Examples.

### [Example 1]

### (Synthesis of TMBN)

TMBN was synthesized according to a synthetic scheme as shown below.

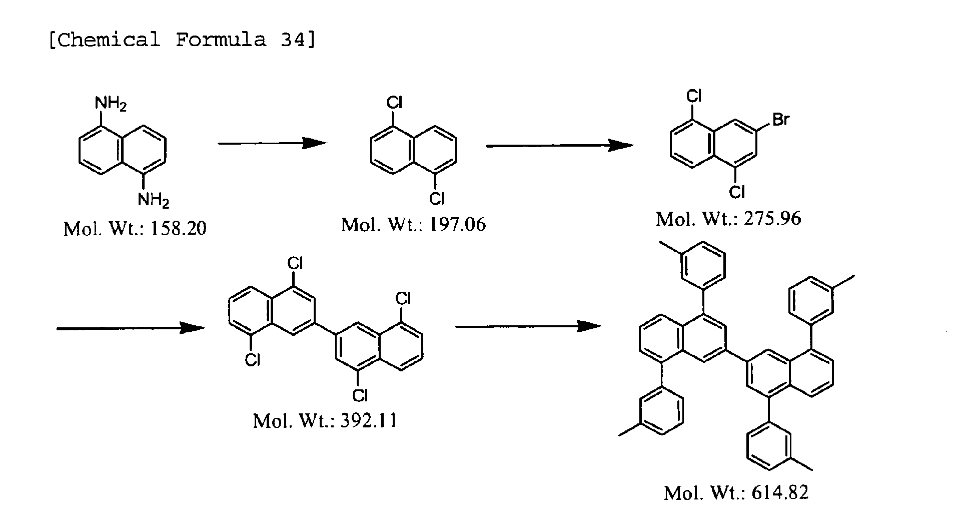

First, 10.4 g (65.7 mmol) of 1,5-diaminonaphthalene was suspended in a sulfuric acid aqueous solution, to which 10.43 g (151.2 mmol) of sodium nitrite in an aqueous solution was added for diazotization under ice-cold conditions. Then, 3 g (50.0 mmol) of urea in an aqueous solution was added to react with excessive sodium nitrite. This solution was added to a concentrated hydrochloric acid solution containing 26.0 g (262.8 mmol) of CuCl to perform chlorination by way of the Sandmeyer reaction under ice-cold conditions.

This solution was stirred at room temperature for 2 to 3 hours, and then deposited solid was filtered and dried.

The thus obtained solid was refined by means of a silica gel column using n-hexane as a developing solvent.

The thus obtained product was identified as 1, 5-dichloronaphthalene, which was the target, as a result of analysis by MS, ¹H-NMR. Its yield was 4.96 g (38.6 % yield).

3.83 g (19.4 mmol) of 1,5-dichloronaphthalene obtained as described above was dissolved in carbon tetrachloride, to which 2.17 g (38.8 mmol) of iron powder was added, then 3.1 g (19.4 mmol) of bromine mixed with carbon tetrachloride was added and reacted overnight.

This reaction solution was treated with a sodium thiosulfate solution, and filtered. The filtrate was washed twice with water and carbon tetrachloride was collected.

The thus obtained solid was refined by means of a silica gel column using a mixed solvent of n-hexane and chloroform as a developing solvent. Further, it was heated in n-hexane, a dibromo derivative was removed by way of hot filtration, and the filtrate was subjected to re-crystallization.

The thus obtained crystal was identified as a bromination derivative, which was the target, as a result of analysis by MS, ¹H-NMR. Its yield was 2.27 g (42.4 % yield).

0.50 g (1.81 mmol) of 3-bromo-1,5-dichloronaphthalene obtained as described above, 0.253g (0.997 mmol) of (pinacolato)diboron, 0.961g (4.53 mmol) of K₃PO₄, and 0.148 g (0.181 mmol) of [Pd(dppf)]Cl₂ were dissolved in dehydrated 1,4-dioxane, which were heated at 90°C in a nitrogen atmosphere for 20 hours.

Water was added to this reaction liquid, and its precipitated crystal was filtered and dried.

The thus obtained solid was refined by means of a silica gel column using chloroform as a developing solvent.

As a result of analysis by MS, ¹H-NMR, the thus obtained crystal was identified as a dimer of 1,5-dichloronaphthalene, which was the target. Its yield was 0.14 g (39.5 % yield).

0.60 g (1.53 mmol) of binaphthyl tetrachloride obtained as described above, 1.66 g (12.2 mmol) of 3-methylphenylboronic acid, 0.0344 g (0.153 mmol) of Pd(OAc)₂, 3.90 g (18.4 mmol) of K₃PO₄, and 0.157 g (0.383 mmol) of 2-dicyclohexylphosphino-2',6'-dimethoxy-biphenyl were dissolved in dehydrated toluene, which were vigorously stirred in a nitrogen atmosphere, at 100°C for one hour to carry out a coupling reaction.

After the reaction, the precipitated crystal was heat dissolved in toluene, and hot filtration of a mineral salt was carried out, then toluene was collected from the filtrate.

The thus obtained solid was refined by means of a silica gel column using a mixed solvent of n-hexane and chloroform as a developing solvent.

As a result of analysis by MS, ¹H-NMR, the thus obtained crystal was identified as TMBN which was the target. Its yield was 0.66 g (70.2 % yield).

Hereafter, TMBN obtained by further subliming and refining the resultant material at 240°C and 2.0×10⁻⁴ Pa was used.

### (Formation of Co-deposition Film of TMBN)

TMBN synthesized as described above was employed as a host material, a co-deposition film in which N2O (maximum wavelength: 436 nm (solution)) allowing blue luminescence was doped by 6 % was formed on a glass board to have a film thickness of 30 nm.

The light emission spectrum was measured about this co-deposition film, and the maximum wave length was 428 nm.

As a result, the above-mentioned co-deposition film gave blue luminescence with high color purity due to N2O, and it was confirmed that TMBN was effective as a host.

### [Example 2]

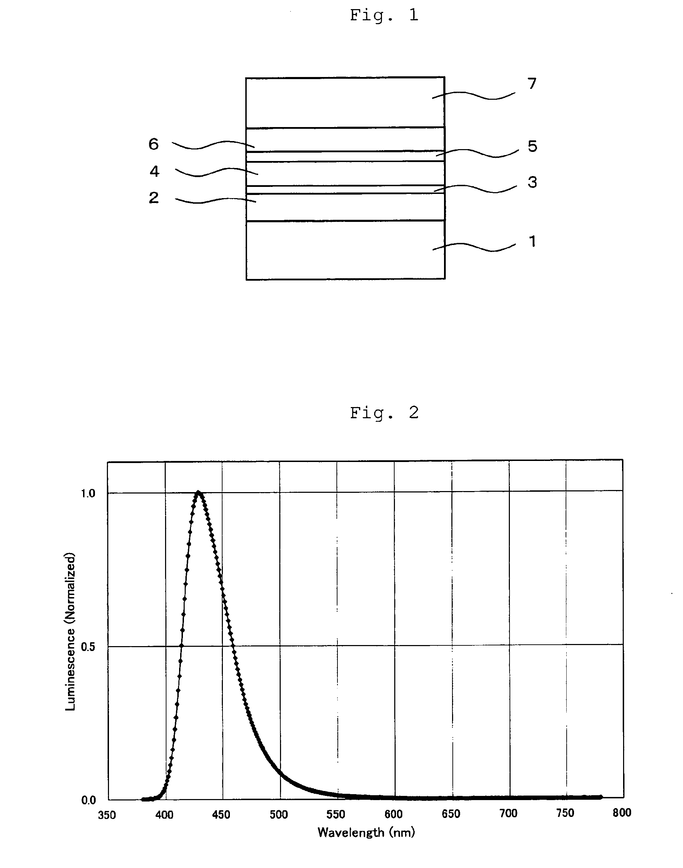

TMBN synthesized as described above was employed as a host material, and an organic EL element having a light emitting layer in which the compound (N20) as shown in the above-mentioned [Chemical Formula 14] was doped, using a compound (DTVPF) as shown in the above-mentioned [Chemical Formula 18] as an organic EL material, and having a layer structure as shown in Fig. 1 was prepared according to the following methods.

### (First Electrode)

First, a glass substrate having formed thereon a patterned transparent electroconductive film (ITO) with a film thickness of 150 nm was subjected to washing treatments in the order of ultrasonic cleaning by pure water and a surfactant, washing with running pure water, ultrasonic cleaning by a 1:1 mixed solution of pure water and isopropyl alcohol, and boiling washing by isopropyl alcohol. This substrate was slowly pulled up from the boiling isopropyl alcohol, and dried in isopropyl alcohol vapor, and, finally ultraviolet ozone cleaning was performed.

This substrate was used as an anode 1 and placed in a vacuum chamber which was evacuated to 1×10⁻⁶ Torr. In this vacuum chamber, each molybdenum boat filled up with a vapor deposition material and a vapor deposition mask for forming a film in a predetermined pattern were placed, the above-mentioned boat was electrically heated, and the vapor deposition material was evaporated to thereby form each organic layer one by one.

### (Hole Injection Transport Layer)

By using the compound (DTVPF) shown in the above-mentioned [Chemical Formula 18] as a hole transport material together with molybdenum trioxide (MoO₃), the respective boats were electrically heated at the same time to carry out co-deposition. A hole injection layer 2 of DTVPF: MoO₃=67:33 was formed to have a film thickness of 10 nm.

Next, a hole transport layer 3 made only of DTVPF was formed to have a film thickness 56 nm.

### (Light Emitting Layer)

A light emitting layer 4 of TMBN:N20=94:6 was formed to have a film thickness of 15 nm.

### (Electron Injection Transport Layer)

An electron transport layer 5 made of DTVPF was formed to have a film thickness of 38 nm, on which an electron injection layer 6 of DTVPF:Liq=50:50 as an electron transport material was formed to have a film thickness of 10 nm.

### (Second Electrode)

While maintaining the vacuum chamber at a vacuum, masks were replaced to install masks for cathode vapor deposition. An aluminum (Al) layer was formed having a film thickness of 100 nm to be a cathode 7.

The vacuum chamber was returned to ambient pressure, and the substrate on which each layer was deposited as described above was taken out, and it was moved in a glove box in which nitrogen substitution was carried out. By using a UV curing resin, it was sealed with another glass board to obtain an organic EL element.

A layer structure of this element may be simplified and shown as being ITO (150 nm) / DTVPF: MoO₃ (10 nm, 67:33) / DTVPF (56 nm) / TMBN:N20 (15 nm, 94:6) / DTVPF (38 nm) / DTVPF:Liq (10 nm, 50:50) / Al (100 nm).

This organic EL element was supplied with a direct current of 100 A/m², then pure blue luminescence due to N20 was obtained, and external quantum efficiency was 0.89 %. Further, a light emission spectrum of this organic EL element is shown in Fig. 2.

Further, the chromaticity of this luminescence color was (x, y)=(0.157, 0.044) in CIE coordinates (100 A/m²), and it was confirmed as being blue luminescence with high color purity.

### [Comparative Example 1]

The compound (TBADN) as shown in the above-mentioned [Chemical Formula 27] was employed as a host material, and an organic EL element having a light emitting layer in which N20 was doped was prepared similarly to Example 2.

A layer structure of this element may be simplified and shown as being ITO (150 nm) / NS21: MoO₃ (59 nm, 90:10) / NS21 (10 nm) / TBADN:N20 (30 nm, 97:3) / BAlq(5 nm) /DPB (16nm) /DPB:Liq (5 nm, 74:26) / Al (100nm).

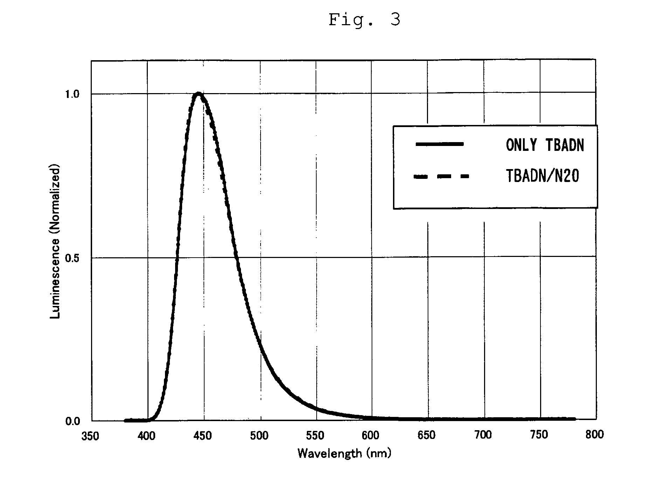

A light emission spectrum of this organic EL element is shown in Fig. 3.

As can be seen from Fig. 3, in the case where TBADN was used as the host, blue luminescence of the N20 origin was not obtained.

Further, the chromaticity of this luminescence color was (x, y)=(0.151, 0.080) in CIE coordinates (100 A/m²), and color purity of blue luminescence was low.

Therefore, it was confirmed that TMBN was excellent as the host compared with TBADN.

As described above, according to the organic EL material expressed by the general formula (1) in accordance with the present invention, the blue luminescence excellent in color purity is obtained, it is confirmed that it is highly practical, and it is expected that it would be applied to a light source and a display apparatus in which excellent color purity is required.

## Claims

1. An organic electroluminescence material expressed by following general formula (1) . 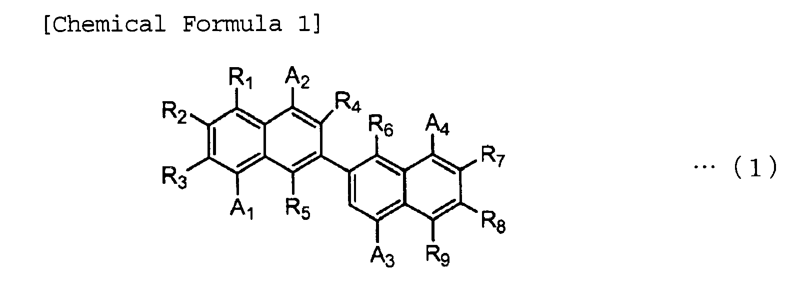 (where R₁-R₉ are selected from the group consisting of hydrogen, an alkyl group, a cycloalkyl group, an alkoxy group, a cycloalkoxy group, and an aryloxy group, and they may be the same groups or the groups different from one another, and A₁-A₄ are selected from the group consisting of a phenyl group which is either substituted or unsubstituted and a 5 or 6 member heterocyclic ring group which is either substituted or unsubstituted, and they may be the same groups or the groups different from one another.)

2. An organic electroluminescence element having one or a plurality of organic layers including a light emitting layer between a pair of electrodes, wherein at least one layer of said organic layers contains the organic electroluminescence material as claimed in claim 1 independently or as a mixture.

3. The organic electroluminescence element as claimed in claim 2, wherein at least one layer of said organic layers is a light emitting layer containing the organic electroluminescence material as a host material as claimed in claim 1 and a fluorescence or phosphorescence emitting material as a guest material.

4. The organic electroluminescence element as claimed in claim 2, wherein said electrode is such that a transparent electroconductive thin-film is formed on a transparent substrate.
